# EUROPEAN PATENT SPECIFICATION

(11) **EP 1 658 870 B1**
(45) Date of publication and mention of the grant of the patent: **23.01.2008**
(21) Application number: 05077681.4
(22) Date of filing: 23.11.2005
(51) Int. Cl.: A61M 5/00, A61M 5/32, A61M 25/06

(54) **Brachytherapy catheter needle**
Katheternadel zur Brachytherapie
Aiguille de cathéter pour curiethérapie

(30) Priority: 23.11.2004 NL 1027562
(43) Date of publication of application: 24.05.2006
(73) Proprietor: Isodose Control Intellectual Property B.V., 3905 PE Veenendaal (NL)
(72) Inventor: van 't Hooft, Eric, 2930 Brasschaat (BE)
(74) Representative: Winckels, Johannes Hubertus F.

(56) References cited:
- US-A- 5 116 323
- US-A1- 2004 193 044

## Description

This invention relates to a brachytherapy catheter needle for inserting a brachytherapy catheter into a part of the body.

In some therapeutic treatments, in particular patient radiation therapy for the treatment of tumors, it is necessary to provide a plurality of brachytherapy catheters in the tissue. For instance in the treatment of breast tumors in women, it is conventional usage to clamp the breast between two templates and, with the breast in compressed condition, to place the brachytherapy catheters via openings provided in the templates. Subsequently, radioactive sources are positioned in the brachytherapy catheters in order to irradiate a tumor present in the breast without unduly burdening the surrounding tissue. For the patient, a treatment with such a device, whereby the breast is flattened, is very unpleasant, often necessitating total anesthesia. A further drawback of the breast being flattened is that the target volume to be treated is enlarged, which necessitates additional needles to enable sufficiently homogeneous irradiation of the target site. In the case of a radiation treatment taking several days, a patient must tolerate the hard flat templates protractedly to avoid a risk of dosimetric errors due to the distance between the needles not having been kept equal.

Further, use is made of conventional brachytherapy catheter needles. Such needles are cylinder-shaped hollow structures which, after placement in the tissue, function as guide tube for a thin guide connected to the brachytherapy catheter. After the thin guide has been wholly arranged in the open needle, needle and brachytherapy catheter are jointly pulled through the tissue, so that the brachytherapy catheter ends up in place. Then the brachytherapy catheter is cut off and a counter stopper is slipped onto it. The cut portion is then measured off and used as coupling tube to connect a guide tube.

The hollow brachytherapy catheter needles are not very strong and moreover rather expensive. The object of the invention is to provide a device whereby a more acceptable treatment with a better dose distribution can be offered, which is simpler to use.

This object is achieved by a device according to the features of claim 1, in particular, a brachytherapy catheter needle, which is formed as an elongate pointed leading part terminating in a coupling piece for fixation of the brachytherapy catheter behind and in line with the leading part. Such a structure is by nature much stiffer, so that the use of a solid template for controlling the direction is necessary to a much lesser extent. In addition, in particular in breast radiation treatment, the brachytherapy catheter needle can be pulled entirely through the tissue, so that the brachytherapy catheter can be cut through at the exit side. This method can be performed faster and more effectively than with the conventional brachytherapy catheter needles. The brachytherapy catheter needle according to the invention is preferably relatively short and solid and can be inserted into the tissue in front of the brachytherapy catheter tube. This provides the advantage that the needle can be made of relatively stiff design, enabling a better straight guidance.

The invention will be further elucidated in and by the description of the drawing.

In the drawing:
Fig. 1 shows a conventional brachytherapy catheter; and
Fig. 2 shows a schematic representation of a brachytherapy catheter according to the invention.

In the Figures, the same or corresponding parts are designated by the same reference numerals.

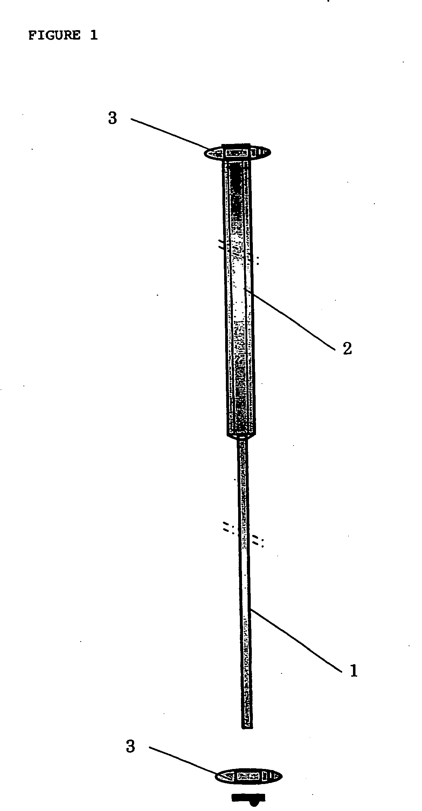

In Fig. 1 a conventional brachytherapy catheter is shown. By means of a hollow needle (not shown) a thin stiff guide wire 1 is introduced into the body, over which a brachytherapy catheter 2 can be pushed into the body. In a treatment in which the needle is pulled entirely through the body, the needle and needle guide are pulled out of the body at the exit side. On the brachytherapy catheter 2, a stop element 3 may be present to prevent the tube sliding into the body too far.

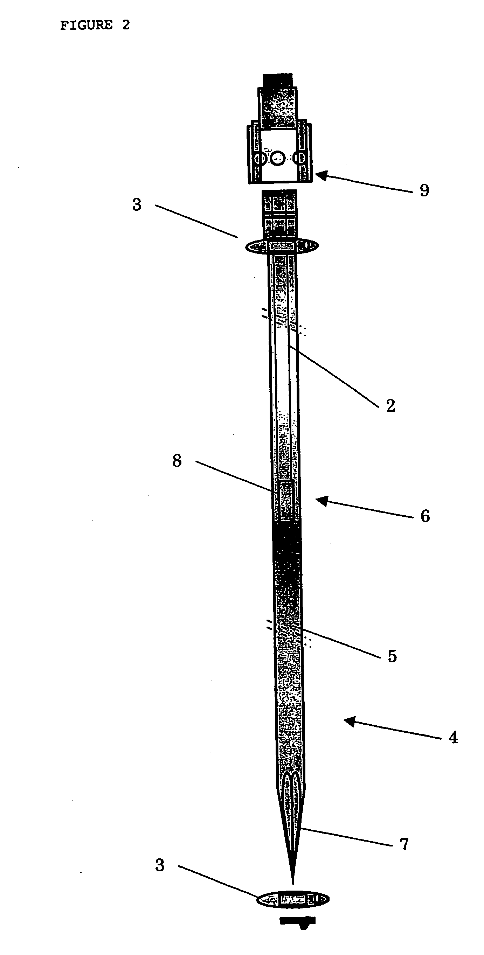

Fig. 2 shows the preferred embodiment of the invention. Here, the brachytherapy catheter needle 4 is formed as an elongate leading part 5 which terminates in a coupling piece 6 for fixation of the brachytherapy catheter 2 behind and in alignment with the leading part 5. In use, this brachytherapy catheter needle 4, in particular in the case of breast radiation treatments, can simply be inserted through the tissue, thereby passing the brachytherapy catheter 2 through the tissue. When the brachytherapy catheter 2 has been pulled through, the needle can be cut off from the brachytherapy catheter 2 at the exit side, eliminating the need to remove the needle via the brachytherapy catheter 2 at the entry side.

By virtue of such a brachytherapy catheter needle 4, a great saving of time can be accomplished when implanting brachytherapy catheters for irradiating tumors, for instance in the female breast. Also, this needle 4 can be made of considerably cheaper design than the conventional brachytherapy catheter needles. The needle is preferably of solid design, so that during insertion it will hardly bend, thereby achieving a high mutual parallelism between brachytherapy catheters. The solid leading part 5 can be provided with a very sharp tip 7. Provided at the side remote from the tip 7 is the coupling piece 6. In particular, the coupling piece 6 is formed by a tailing part protruding from the needle body, for insertion in a brachytherapy Catheter 2, the coupling piece 6 being smaller in diameter than the diameter of the needle 4. In this example the coupling part forms a squeeze fitting 8 with brachytherapy Catheter 2. However, the coupling part can also be formed by a bracket which encloses the brachytherapy Catheter. Accordingly the brachytherapy catheter 2 is coupled behind and in line with the brachytherapy catheter needle 4 and a smooth transition between needle 4 and brachytherapy catheter 2 is achieved. In particular, the needle diameter is larger than the diameter of the brachytherapy catheter 2. Fig. 2 further shows schematically that the brachytherapy catheter 2 is coupled with a coupling element 9 of a radiation dosing device (not shown). What can be achieved through stop elements 3 which are slipped over the brachytherapy catheter 2 or are fixedly fitted thereon is that the brachytherapy catheter 2 is fixed in the tissue and axial shifts are avoided, by stopping the brachytherapy catheter at the front and/or the back.

The invention has been elucidated on the basis of the preferred embodiment. It is to be understood here that other embodiments falling within the scope of the invention are also possible. For instance, the brachytherapy catheter can be straight or curved and used with or without template.

## Claims

1. A brachytherapy catheter needle (4) for inserting a brachytherapy catheter into a part of the body, **characterized in that**
- the brachytherapy catheter needle is formed as an elongate pointed leading part (5) which terminates in a coupling piece (6), the coupling piece (6) adapted for coupling and fixation of the brachytherapy catheter (2) to the brachytherapy needle (4), the coupling piece arranged as to be behind and in line with the leading part of the brachytherapy needle.

2. A brachytherapy catheter needle (4) according to claim 1, **characterized in that** the leading part is of solid design.

3. A brachytherapy catheter needle (4) according to claim 1, wherein a needle diameter is larger or equal than brachytherapy catheter diameter.

4. A brachytherapy catheter needle (4) according to claim 1, wherein the coupling piece (6) is smaller in diameter than the needle diameter for insertion in a brachytherapy catheter, for providing a smooth transition between needle and brachytherapy catheter (2).

5. A brachytherapy catheter needle (4) according to claim 1, **characterized in that** the coupling piece (6) comprises a squeeze fitting.

6. A brachytherapy catheter needle (4) according to at least one of the preceding claims, **characterized in that** the brachytherapy catheter needle (4) comprises a stopper for stopping the brachytherapy catheter at the front and/or back.

7. A brachytherapy catheter needle (4) according to at least one of the preceding claims, **characterized in that** it is fixedly connected with the brachytherapy catheter (2).

8. A brachytherapy catheter needle (4) according to claim 7, **characterized in that** the brachytherapy catheter comprises a fixed stop element (3).

## Patentansprüche

1. Brachytherapiekatheternadel (4) zum Einführen eines Brachytherapiekatheters in ein Körperteil, **dadurch gekennzeichnet, dass**
die Brachytherapiekatheternadel als längliches, spitzes führendes Teil (5) gebildet ist, das in einem Kopplungsstück (6) endet, wobei das Kopplungsstück (6) dazu ausgebildet ist, den Brachytherapiekatheter (2) an der Brachytherapienadel (4) anzubringen und zu fixieren, wobei das Kopplungsstück dazu ausgebildet ist, hinter und in Ausrichtung mit dem führenden Teil der Brachytherapienadel zu sein.

2. Brachytherapiekatheternadel (4) gemäß Anspruch 1, **dadurch gekennzeichnet, dass** das führende Teil von fester Gestaltung ist.

3. Brachytherapiekatheternadel (4) nach Anspruch 1, wobei ein Nadeldurchmesser größer als oder gleich dem Brachytherapiekatheterdurchmesser ist.

4. Brachytherapiekatheternadel (4) nach Anspruch 1, wobei das Kopplungsstück (6) im Durchmesser kleiner als der Nadeldurchmesser für das Einführen in einen Brachytherapiekatheter ist, um einen glatten Übergang zwischen der Nadel und dem Brachytherapiekatheter (2) bereitzustellen.

5. Brachytherapiekatheternadel (4) nach Anspruch 1, **dadurch gekennzeichnet, dass** das Kopplungsstück (6) eine Klemmpassung aufweist.

6. Brachytherapiekatheternadel (4) nach wenigstens einem der vorhergehenden Ansprüche, **dadurch gekennzeichnet, dass** die Brachytherapiekatheternadel (4) einen Stopper aufweist, um den Brachytherapiekatheter vorne oder hinten zu stoppen.

7. Brachytherapiekatheternadel (4) nach wenigstens einem der vorhergehenden Ansprüche, **dadurch gekennzeichnet, dass** sie fest mit dem Brachytherapiekatheter (2) verbunden ist.

8. Brachytherapiekatheternadel (4) nach Anspruch 7, **dadurch gekennzeichnet, dass** der Brachytherapiekatheter ein festes Stoppelement (3) aufweist.

## Revendications

1. Aiguille de cathéter de curiethérapie (4) pour insertion d'un cathéter de curiethérapie dans une partie du corps, **caractérisée en ce que**
- l'aiguille de cathéter de curiethérapie est formée sous la forme d'une partie d'attaque pointue allongée (5) qui se termine en une pièce de couplage (6), la pièce de couplage (6) étant adaptée pour couplage et fixation du cathéter de curiethérapie (2) sur l'aiguille de curiethérapie (4), la pièce de couplage étant prévue de manière à être derrière la partie d'attaque de l'aiguille de curiethérapie, et alignée avec celle-ci.

2. Aiguille de cathéter de curiethérapie (4) selon la revendication 1, **caractérisée en ce que** la partie d'attaque est d'une conception pleine.

3. Aiguille de cathéter de curiethérapie (4) selon la revendication 1, dans laquelle le diamètre d'aiguille est supérieur ou égal au diamètre de cathéter de curiethérapie.

4. Aiguille de cathéter de curiethérapie (4) selon la revendication 1, dans laquelle la pièce de couplage (6) est un diamètre plus petit que le diamètre d'aiguille pour insertion dans un cathéter de curiethérapie, afin de fournir une transition régulière entre l'aiguille et le cathéter de curiethérapie (2).

5. Aiguille de cathéter de curiethérapie (4) selon la revendication 1, **caractérisée en ce que** la pièce de couplage (6) comporte un raccord comprimable.

6. Aiguille de cathéter de curiethérapie (4) selon l'une quelconque des revendications précédentes, **caractérisée en ce que** l'aiguille de cathéter de curiethérapie (4) comporte une butée pour arrêt du cathéter de curiethérapie à l'avant et/ou à l'arrière.

7. Aiguille de cathéter de curiethérapie (4) selon l'une quelconque des revendications précédentes, **caractérisée en ce qu'**elle est reliée de manière fixe au cathéter de curiethérapie (2).

8. Aiguille de cathéter de curiethérapie (4) selon la revendication7, **caractérisée en ce que** le cathéter de curiethérapie comporte un élément d'arrêt fixe (3).
